# EUROPEAN PATENT SPECIFICATION

(11) **EP 0 689 461 B1**
(45) Date of publication and mention of the grant of the patent: **05.07.2000**
(21) Application number: 94909226.6
(22) Date of filing: 16.03.1994
(51) Int. Cl.: A61K 49/00

(54) **IMPROVEMENTS IN OR RELATING TO CONTRAST AGENTS**
VERBESSERUNGEN AN UND IN BEZIEHUNG ZU KONTRASTMITTELN
PERFECTIONNEMENTS APPORTES AUX AGENTS DE CONTRASTE

(30) Priority: 16.03.1993 GB 9305349
(43) Date of publication of application: 03.01.1996
(73) Proprietor: NYCOMED IMAGING AS, 0401 Oslo 4 (NO)
(72) Inventor: BERG, Arne, N-1300 Sandvika (NO); DUGSTAD, Harald, N-0489 Oslo (NO); FOSS, Per, Antonius, N-0377 Oslo (NO); KLAVENESS, Jo, N-1166 Oslo (NO); OSTENSEN, Jonny, N-1053 Oslo (NO); RONGVED, Pal, N-1450 Nesoddtangen (NO); STRANDE, Per, N-0755 Oslo (NO)
(74) Representative: Marsden, John Christopher
(86) International application number: GB9400521
(87) International publication number: WO9421301

(56) References cited:
- EP-A- 0 245 019
- EP-A- 0 554 213
- WO-A-93/05819
- US-A- 4 684 479
- US-A- 4 900 540

## Description

This invention relates to novel contrast agents, more particularly to new contrast agents of use in diagnostic ultrasound imaging.

Ultrasound imaging is based on penetration of ultrasound waves, e.g. in the frequency range 1-10 MHz, into a human or animal subject via a transducer, the ultrasound waves interacting with interfaces of body tissues and fluids. Contrast in an ultrasound image derives from differential reflection/absorption of the sound waves at such interfaces; results may be enhanced by the use of Doppler techniques, including the use of colour Doppler to evaluate blood flow.

It has long been realised that it may be advantageous to increase the difference in acoustic properties of different tissues/fluids using contrast agents, and since the use of indocyanine green in 1968 as the first ultrasound contrast agent many other potential agents have been examined. These include emulsions, solid particles, water-soluble compounds, free gas bubbles and various types of encapsulated gas-containing systems. It is generally accepted that low density contrast agents which are easily compressible are particularly efficient in terms of the acoustic backscatter they generate; gas-containing and gas-generating systems thus tend to exhibit markedly greater efficacy than other types of contrast agent.

Three ultrasound contrast agents are now commercially available or in late clinical development, these being Echovist®, based on gas-containing galactose microcrystals; Levovist®, comprising gas-containing galactose microcrystals coated with fatty acid; and Albunex®, which comprises gas bubbles encapsulated by partially denatured human serum albumin. Clinical use of these agents is restricted, however, by their short contrast half-lives (i.e. by their relative lack of stability in vivo) and their limited shelf life. Accordingly there is a continuing need for ultrasound contrast agents, especially for cardiac and non-cardiac perfusion studies, which combine good storage stability with stability in vivo, preferably for at least several passages of circulation in the case of intracardiac injections.

A further disadvantage of microparticulate ultrasound contrast agents such as Echovist® and Levovist® is that they need to be formulated prior to administration, e.g. by addition of an appropriate carrier liquid and agitation by shaking. This inevitably causes some delay and there is thus a need for improved ultrasound contrast agents which can be stored for substantial periods of time (e.g. at least 12 months, preferably 2-3 years) in "ready to use" form.

The present invention is based on our findings that this objective may be fulfilled by oil-in-water emulsion-based contrast agents containing oil-soluble gases/fluids or gas precursors in condensed or dissolved form in the dispersed oil phase.

Thus according to one feature of the invention there is provided an ultrasound contrast agent consisting of a biocompatible oil-in-water emulsion and any necessary biocompatible emulsification agent or agents, the oil phase of said emulsion consisting essentially of a solution in a lipophilic solvent of an oil-soluble and substantially water-insoluble material, elected from gases, volatile fluids and gas precursors.

A characteristic feature of the contrast agents of the invention is that they are substantially completely free from gas bubbles/microbubbles in their stored form prior to administration; rapid microbubble generation may ensue following administration, however, e.g. by intravenous or intra-arterial injection, or may be induced immediately before such administration, e.g. as described in greater detail hereinafter. In this respect the products of the invention may be contrasted with existing ultrasound contrast agents, which in general contain free gas in their stored form, e.g. as inclusions in the voids of their crystal structures and/ or adhered to their surfaces in the case of micro-particulate materials such as Echovist® and Levovist® or in encapsulated form in materials such as Albunex®.

Gases of use in the contrast agents of the invention preferably have low water solubility to ensure their preferential solubility in the lipophilic oil phase of the emulsion and to enhance the stability of the microbubbles generated in the aqueous environment of the bloodstream following intravenous or intra-arterial injection of the contrast agents. It will be appreciated, however, that the solubility of the gas in the oil phase should preferably not be so high as to inhibit such microbubble generation following administration, although microbubble formation may if desired be enhanced in such situations by, for example, preheating the emulsion prior to administration, e.g. as described in greater detail hereinafter. Appropriate gases thus include, for example, inert gases such as helium, neon, argon, krypton or xenon; hydrocarbons such as methane, acetylene or 3-methylbut-l-ene; halogenated hydrocarbons, including haloalkanes such as methyl bromide, C₁₋₄ hydrofluoroalkanes such as hexafluoropropane, and, more preferably, perfluoroalkanes such as perfluoromethane or perfluorobutane and sulphur halides such as sulphur hexafluoride etc.

The term "gas" as used herein includes any substance in the gaseous form at the normal human body temperature of 37°C, and thus embraces a variety of oil-soluble substances which are liquid at ambient temperatures, e.g. 20-25°C. Examples of suitable substances, which may be regarded as being intermixed with the oil phase of the emulsion in the context of being dissolved therein, include optionally halogenated and/or otherwise substituted hydrocarbons, aliphatic and cyclic ethers, silanes, arsines and sulphur halides, for example such as the following:

| Substance | boiling point (°C) |
|---|---|
| 4-methyl-1,3-dioxolan-2-one | 24.2 |
| dibromodifluoromethane | 24.5 |
| 1-nitroheptafluoropentane | 25.0 |
| tetramethylsilane | 26.5 |
| but-2-yne | 27.0 |
| 2-methylbutane | 27.8 |
| disulphur decafluoride | 29.0 |
| perfluoropent-l-ene | 29-30 |
| pent-l-ene | 30.0 |
| 1,2-difluoroethane | 30.7 |
| 2-methylbut-l-ene | 31.2 |
| furan | 31.4 |
| n-butyl fluoride | 32.5 |
| methyl isopropyl ether | 32.5 |
| tris-trifluoromethylarsine | 33.3 |
| 2-methylbuta-1,3-diene | 34.1 |
| propylene oxide | 34.2 |
| diethyl ether | 34.5 |
| isopropyl chloride | 35-36 |
| pentane | 36.1 |
| pent-2-ene (trans, cis) | 36.3-36.9 |

The term "fluid" as used herein denotes a volatile organic substance, preferably having a boiling point not exceeding 60°C. It will be appreciated that the above-described requirements of low water solubility and preferential (but desirably not excessive) oil solubility as applied to such fluids should be interpreted as ones of miscibility and that the requirement of dissolution in the oil phase of the emulsion should be interpreted as one of being intermixed therewith.

Examples of fluids include 1,1-dichloroethylene, 2-methylbut-2-ene, 3,3-dimethylbut-l-yne, dimethylaminoacetone, perfluoropentane, cyclopentane, cyclopentene and 2,3-pentadiene. It will be appreciated that such fluids having boiling points in excess of 37°C will in general not generate gas microbubbles following administration. They will, however, generate fluid microbubbles which by virtue of the relatively low density of the fluid will provide an ultrasound contrast effect.

Examples of oil-soluble gas precursors include organic carbonates, e.g. compounds of formula

RO.CO.OM

where R is a lipophilic organic group and M is a physiologically acceptable cation. Such substances will generate carbon dioxide at pHs of about 7 or less, e.g. under the conditions prevailing in the bloodstream following intravenous or intra-arterial administration. Where such precursors relying on pH activation are employed it may be advantageous to incorporate an ionophore, e.g. nigericin, into the emulsion to facilitate proton transfer through the oil phase.

Other gas precursors include nitrogen-generating substances such as pyrazolines, pyrazoles, triazolines, diazoketones, diazonium salts, tetrazoles, azides and azide/carbonate mixtures, which may, for example, be activated by irradiation, e.g. by UV light, for example immediately prior to administration. Substances which generate carbon dioxide upon photolysis, e.g. certain cyclic ketones, lactones and carbonates, may similarly be useful.

Oxygen-generating gas precursors include peracids such as perbenzoic acid.

Thermally degradable gas precursors, which are activated by body heat following administration, may also be used, an example of such a substance being a thermally degradable carboxylic acid such as 2-methyllactic acid.

A further class of gas precursors comprises substances which are enzymically degraded in vivo with accompanying generation of gas. Examples include methylene diesters (e.g. prepared using techniques such as are described in WO-A-9317718 and WO-A-9318070, the contents of which are incorporated herein by reference), which are cleaved by common esterases leading to evolution of carbon dioxide. Another useful substance is hydrogen peroxide, which is soluble in lipophilic media such as ethers and which is enzymatically degraded in vivo with evolution of oxygen. If hydrogen peroxide is used it may be advantageous also to incorporate an antioxidant stabiliser.

One useful class of lipophilic solvent components for the oil phase of emulsions according to the invention comprises highly fluorinated organic compounds such as have been proposed as components of "artificial bloods" - see for example EP-A-0231091 and WO-A-8910118, the contents of which are incorporated herein by reference. It should be noted that when used in artificial bloods these fluorinated compounds are believed to effect oxygen transport by complexing with oxygen molecules, in contrast to the present invention where they effectively provide a solvent medium for the lipid-soluble gas/fluid.

Highly fluorinated organic compounds which may be used in accordance with the invention include aliphatic and cycloaliphatic perfluorocarbons, e.g. containing up to 20 carbon atoms, such as perfluoro-2,2,4,4-tetra-methylpentane, perfluorooctane, perfluorodecane, perfluorotrimethylcyclohexane, perfluoroisopropylcyclohexane, perfluorodecalin, perfluoroindane, perfluorotrimethylbicyclo[3.3.1]nonane, perfluorobicyclo[5.3.0]decane, perfluoromethyladamantane and perfluorodimethyladamantane; bromo-substituted analogues of the foregoing, such as perfluororoctyl bromide; aliphatic and cycloaliphatic perfluoroamines, e.g. containing up to 20 carbon atoms, such as perfluorotripropylamine, perfluorotributylamine, perfluoro-N-methyldecahydroquinoline, perfluoro-4-methyloctahydroquinolizidine and perfluoro-1-azatricyclic amines; perfluoro ethers and mixtures of any of the foregoing. Preferred compounds of this type include perfluorooctyl bromide and perfluorodecalin, which latter may for example be used in combination with perfluorotripropylamine, e.g. as in the product Fluosol DA®.

Biocompatible emulsification agents which may be used in the contrast agents of the invention include surfactants and other stabilisers. It will be appreciated that such emulsification agents may be preferentially dissolved or dispersed in either the oil phase or the aqueous phase as needed for specific emulsion systems. Appropriate biocompatible surfactants which may be useful thus include anionic surfactants, tor example alkali metal salts of fatty acids such as sodium dodecanoate, alkali metal alkyl sulphates such as sodium lauryl sulphate and alkali metal sulphonated esters such as sodium dioctyl sulphosuccinate (docusate) and, more preferably, nonionic or zwitterionic surfactants. Examples of these latter categories include fatty alcohols such as n-decanol, polyoxyethylene-polyoxypropylene copolymers (e.g. poloxamers such as Pluronic® F68), sorbitan fatty acid esters such as Span-type materials and polyoxyethylated analogues thereof such as Tween-type materials, phospholipids (e.g. phosphatidyl choline (i.e. lecithin) or dioleoylphosphatidyl diethanolamine), and polyethylene glycol esters of fatty acids (e.g. Cremaphor® products).

Polymerisable amphiphiles, for example such as those described in WO-A-9217212 (the contents of which are incorporated herein by reference), may also be used as surfactants; polymerisation of such amphiphiles, e.g. by UV-irradiation or other appropriate form of initiation, may if desired be effected after emulsification.

Appropriate surfactants may be employed in the form of multicompartment vesicles, e.g. as described by Kim et al. in Biochim. Biophys. Acta 728 (1983) p. 339 and in EP-A-0280503. These may be regarded as consisting of lipid bilayer membranes enclosing a plurality of non-concentric cores, i.e. having a quasi-honeycomb structure. A plurality of amphipathic lipids may conveniently be used, at least one of these advantageously having a net negative charge; one or more neutral lipids may also be present. Representative components thus include phosphatidyl serines, phosphatidylglycerols such as dimyristoyl phosphatidylglycerol, phosphatidic acids such as dimyristoyl phosphatidic acid, phosphatidyl cholines such as dioleoylphosphatidyl choline or dipalmitoylphosphatidyl choline, phosphatidyl ethanolamines, dioleoyl lecithin, cholesterol, triolein, trioctanoin and other oils/ triglycerides and derivatives thereof.

Other lipophilic substances which may be used in the emulsions, e.g. as stabilising components, include antioxidants such as tocopherols or thioctic acid, perfluorinated surfactants which both dissolve and stabilise the lipid-soluble gas or gas precursor, liquid crystals, compounds for making Langmuir-Blodget films, and lipophilic biodegradable polymers, for example block copolymers, (e.g. as described in WO-A-9204392 or WO-A-9317718). Oil-soluble carrier molecules for the gas/fluid may also be employed; porphyrins may be suitable carriers for this purpose.

Additives such as surfactant assistants may also be employed, for example viscosity enhancers such as sugars, e.g. sucrose.

One particularly useful class of emulsions comprises fat-based emulsions such as the commercially available intravenously administrable emulsions Liposyn® (Abbot Laboratories), Intralipid® (Kabi Vitrum) and Soyacal® (Alpha Therapeutic). Such emulsions are typically based on soyabean oil, egg yolk phospholipid, glycerin and water for injection, and typically have emulsion particles less than 0.5 µm in diameter, similar in size to naturally occurring chylomicrons. Advantages of their use may include long shelf life, improved vascular contrast half-life and sustained release of gas.

If desired, the oil phase of contrast agents according to the invention may additionally contain suspended solid microparticles of one or more biotolerable minerals, e.g. having a particle size of less than 1 micron, preferably less than 0.2 microns. Such microparticles, which may for example comprise silica or iron oxide, may act as nucleation sites, promoting generation of gas at the solid/liquid interface following administration of the contrast agents.

The precise constitution of contrast agents according to the invention may be varied widely depending on such factors as the particular components used, the specific usage envisaged and the intended microbubble size following administration.

Thus, for example, the size of microbubbles formed following administration will generally increase as the concentration of gas/fluid is increased, also being affected by the nature of the materials forming the oil phase. Where the contrast agent contains a dissolved gas this may in general be at any desired level up to saturation or even supersaturation, e.g. with the contrast agent being stored under pressure.

In the case of pressurised contrast agents microbubble formation may commence before administration of the agent, e.g. as soon as the vial or other form of container is broached, and will continue in vivo following administration. Non-pressurised contrast agents will generate microbubbles in vivo as a result of, for example, warming of the contrast agent to body temperature, diffusion of blood components into the stabilising material and/or gradual breakdown of the emulsion. Alternatively, microbubble generation may be induced prior to administration, for example by preheating the emulsion, e.g. by microwave heating.

As noted above, preferred oil-soluble gases are those having low solubility in water. This encourages the gas to associate with lipophilic components of the emulsion, thereby further enhancing the stability of the microbubbles, and may also lead to generation of a flexible microbubble/lipophile matrix, e.g. in the form of coated microbubbles. It is recognised in the art that such flexible structures are particularly advantageous by virtue of their enhanced ultrasound contrast effect when compared to more rigid encapsulated microbubble systems.

Contrast agents according to the invention may be prepared by any convenient method. Thus, for example, a gas, volatile fluid-containing or gas precursor-containing oil phase, e.g. consisting of a solution of an oil-soluble gas, volatile fluid or gas precursor in at least one lipophilic solvent medium, may be emulsified in an aqueous phase so as to form an oil-in-water emulsion, e.g. using conventional techniques such as homogenisation or sonication, or the desired gas/fluid or gas precursor may be incorporated into the oil phase of a preformed oil-in-water emulsion.

Where an oil-soluble gas is employed this may, for example, be dissolved in a chosen lipophilic solvent medium, e.g. at elevated pressure, the resulting oil thereinafter being emulsified, advantageously under a pressure of excess gas and, if necessary or desired, in the presence of one or more biocompatible emulsifiers. Analogous techniques may be used when an oil-soluble fluid or gas precursor is employed. Alternatively, gas may be incorporated into a preformed emulsion, for example by passing gas through the emulsion and/or by maintaining the emulsion under an elevated pressure of gas.

The ultrasound contrast agents of the invention may, for example, be administered enterally or parenterally, although there may be advantages in particular applications in administration directly into body cavities such as the Fallopian tubes. In general, however, intravascular administration, most commonly by intravenous injection, is most likely to be employed, in order to enhance vascular imaging, including cardiac and extracardiac perfusion.

It will be appreciated that contrast agents for intravenous administration should generate microbubbles small enough to pass through the capillary bed of the pulmonary system. The agents should therefore preferably be such as to generate microbubbles having diameters of less than 10 µm, preferably in the range 0.2-8 µm, e.g. 0.3-7 µm.

The following non-limitative examples serve to illustrate the invention.

### EXAMPLE 1

Span 20 (0.10 g) was dispersed in perfluorodecalin (4 ml) which then was saturated with sulphur hexafluoride at 4°C. Tween 60 (0.45 g) was dissolved in water (36 ml), cooled to 4°C and the two solutions were emulsified at 4°C using an Ystral homogenizer at 20 000 rpm for 30 seconds. Ultrasound attenuation was measured by injecting 2 ml of the emulsion into 6 ml of distilled water at 4°C and heating slowly to 37°C. An attenuation of 2 dB/cm or higher was observed for approximately 120 seconds.

### EXAMPLE 2

n-Decanol (0.5 ml) and sodium dodecanoate (0.50 g) were dispersed in water (36 ml). Perfluorodecalin (4 ml) was cooled to 4°C and saturated with sulphur hexafluoride. The two solutions were emulsified at 4°C using an Ystral homogenizer at 20 000 rpm for 30 seconds. Ultrasound attenuation was measured by mixing 2 ml of the emulsion with 6 ml of distilled water at 37°C. A strong attenuation (> 2 dB/cm) was observed for approximately 30 seconds.

### EXAMPLE 3

The emulsion from Example 2 (2 ml) was mixed with distilled water (6 ml) at 4°C and the diluted emulsion was slowly heated to 37°C. The ultrasound attenuation was measured and a maximum attenuation of 2.7 dB/cm was observed after approximately 30 seconds.

### EXAMPLE 4

n-Decanol (0.5 ml) and sodium dodecanoate (0.50 g) were dispersed in water (36 ml). Perfluorodecalin (4 ml) was cooled to 4°C and saturated with xenon. The two solutions were emulsified at 4°C using an Ystral homogenizer at 20 000 rpm for 30 seconds. Ultrasound attenuation was measured by mixing 2 ml of the emulsion with 6 ml of distilled water at 37°C. The ultrasound attenuation increased from 1 to approximately 3 dB/cm over 5 minutes.

### EXAMPLE 5

n-Decanol (0.5 ml) and sodium dodecanoate (0.50 g) are dispersed in water (36 ml). Perfluorooctyl bromide (4 ml) is cooled to 4°C and saturated with xenon. The two solutions are emulsified at 4°C using an Ystral homogenizer at 20 000 rpm for 30 seconds. Ultrasound attenuation is measured by mixing 2 ml of the emulsion with 6 ml of distilled water at 37°C.

### EXAMPLE 6

Intralipid® (Kabi Vitrum, Stockholm, Sweden), Fluosol® (Alpha Therapeutic Ltd, UK) or perfluorooctyl bromide (10 ml) is cooled to 4°C in an autoclave. The emulsions are stirred while being pressurised with xenon (20 atm) for 16 hours. The stirring is then stopped and the pressure slowly released. Ultrasound attenuation is measured by mixing 2 ml of each emulsion with 6 ml of distilled water at 37°C.

### EXAMPLE 7

Perfluoro-n-butane (1.6g) at a pressure of 2.5 atmospheres was added to perfluorodecalin (0.4 g) cooled to -5°C, this being below the boiling point of perfluoro-n-butane (ca. -2°C). The resulting oil was emulsified with 40 ml of an aqueous solution containing Pluronic F68 (1% w/w) and sucrose (30% w/w) by sonication for 20 minutes in a cooled closed plastic vessel. Droplet diameter for the thus-obtained oil-in-water emulsion was observed microscopically to be about 1 micron; no significant change was evident after storage for one week. A portion (5 ml) of the emulsion in a 15 ml vessel was heated to 80°C in an 800 W microwave oven (typical heating time 8-10 seconds), cooled and filtered (Millipore, 10 µm). Microscopy confirmed the formation of perfluoro-n-butane microbubbles having stabilising coatings of perfluorodecalin. The in vitro acoustic attenuation for such a microbubble dispersion diluted to have a total oil content of 0.2% w/w was greater than 10 dB/cm over the frequency range 1-6 MHz and was stable for more than 10 minutes. A similar sample prepared without preheating the emulsion and tested at 37°C exhibited an in vitro acoustic attenuation which increased from 2 dB/cm to 6 dB/cm over 7 minutes as microbubbles were generated spontaneously.

## Claims

1. An ultrasound contrast agent consisting of a biocompatible oil-in-water emulsion and any necessary biocompatible emulsification agent or agents, the oil phase of said emulsion consisting essentially of a solution in a lipophilic solvent of an oil-soluble and substantially water-insoluble material, selected from gases, volatile fluids and gas precursors.

2. A contrast agent as claimed in claim 1 wherein the gas or volatile fluid is selected from inert gases, optionally halogenated or otherwise substituted hydrocarbons, aliphatic or cyclic ethers, silanes, arsines and sulphur halides.

3. A contrast agent as claimed in claim 2 wherein the gas or volatile fluid is a perfluoroalkane.

4. A contrast agent as claimed in claim 2 wherein the gas or volatile fluid is selected from xenon, n-pentane, furan, tetramethylsilane, sulphur hexafluoride and perfluoro-n-butane.

5. A contrast agent as claimed in claim 1 wherein the gas precursor is an organic carbonate; a pyrazoline, pyrazole, triazoline, diazoketone, diazonium salt, tetrazole, azide or azide/carbonate mixture; a photolysable cyclic ketone, lactone or carbonate; a peracid; a thermally degradable carboxylic acid; an enzymically degradable methylene diester or hydrogen peroxide.

6. A contrast agent as claimed in claim 1 wherein the lipophilic solvent is selected from aliphatic and cycloaliphatic perfluorocarbons and bromo-substituted analogues thereof, aliphatic and cycloaliphatic perfluoroamines, perfluoro ethers and mixtures of any of the foregoing.

7. A contrast agent as claimed in claim 6 wherein the lipophilic solvent is perfluorooctyl bromide, perfluorodecalin or a mixture of perfluorodecalin and perfluorotripropylamine.

8. A contrast agent as claimed in any of claims 1 to 7 containing as emulsification agent one or more surfactants selected from alkali metal salts of fatty acids, alkali metal alkyl sulphates and sulphonated esters, polyoxyethylene-polyoxypropylene copolymers, sorbitan fatty acid esters and polyoxyethylated analogues thereof, phospholipids, polyethylene glycol esters of fatty acids, polymerisable amphiphiles and multicompartment vesicle surfactant systems.

9. A contrast agent as claimed in any of claims 1 to 5 wherein the oil phase of the emulsion comprises soyabean oil and egg yolk phospholipid and the aqueous phase comprises glycerin and water for injection.

10. A contrast agent as claimed in any of the preceding claims wherein microparticles of one or more biotolerable minerals are suspended in the oil phase.

11. A process for the preparation of an ultrasound contrast agent as defined in claim 1 which comprises either (i) emulsifying an oil phase comprising a solution of an oil-soluble and substantially water-insoluble gas, volatile fluid or gas precursor in at least one lipophilic solvent component in an aqueous phase, if necessary in the pressure of a biocompatible emulsification agent, so as to form an oil-in-water emulsion or (ii) incorporating a desired oil-soluble and substantially water-insoluble gas, volatile fluid or gas precursor into the oil phase of a preformed oil-in-water emulsion.

12. A method of generating enhanced images of a human or non-human animal body previously administered with a contrast agent as claimed in any one of claims 1 to 10, which method comprises generating an ultrasound image of at least part of said body.

## Patentansprüche

1. Ultraschallkontrastmittel, bestehend aus einer biokompatiblen Öl-in-Wasser-Emulsion und, soweit erforderlich, einem oder mehreren biokompatiblen Emulgiermitteln, wobei die Ölphase der Emulsion im Wesentlichen aus einer Lösung eines öllöslichen und im Wesentlichen wasserunlöslichen Materials, das unter Gasen, flüchtigen Flüssigkeiten und Gasvorläufern ausgewählt ist, in einem lipophilen Lösungsmittel besteht.

2. Kontrastmittel nach Anspruch 1, wobei das Gas oder die flüchtige Flüssigkeit unter Inertgasen, gegebenenfalls halogenierten oder auf andere Weise substituierten Kohlenwasserstoffen, aliphatischen oder cyclischen Ethern, Silanen, Arsinen und Schwefelhalogeniden ausgewählt ist.

3. Kontrastmitteln nach Anspruch 2, wobei es sich bei dem Gas oder der flüchtigen Flüssigkeit um ein Perfluoralkan handelt.

4. Kontrastmittel nach Anspruch 2, wobei das Gas oder die flüchtige Flüssigkeit unter Xenon, n-Pentan, Furan, Tetramethylsilan, Schwefelhexafluorid und Perfluor-n-butan ausgewählt ist.

5. Kontrastmittel nach Anspruch 1, wobei es sich bei dem Gasvorläufer um ein organisches Carbonat; ein Pyrazolin, Pyrazol, Triazolin, Diazoketon, Diazoniumsalz, Tetrazol, Azid oder ein Azid/Carbonat-Gemisch; ein photolysierbares cyclisches Keton, Lacton oder Carbonat; eine Persäure; eine thermisch zersetzbare Carbonsäure; einen enzymatisch abbaubaren Methylendiester oder Wasserstoffperoxid handelt.

6. Kontrastmittel nach Anspruch 1, wobei das lipophile Lösungsmittel unter aliphatischen und cycloaliphatischen Perfluorkohlenwasserstoffen und Brom-substituierte Analoga davon, aliphatischen oder cycloaliphatischen Perfluoraminen, Perfluorethern und beliebigen Gemischen davon ausgewählt ist.

7. Kontrastmittel nach Anspruch 6, wobei es sich bei dem lipophilen Lösungsmittel um Perfluoroctylbromid, Perfluordecalin oder ein Gemisch von Perfluordecalin und Perfluortripropylamin handelt.

8. Kontrastmittel nach einem der Ansprüche 1 bis 7, enthaltend als Emulgiermittel ein oder mehrere unter Alkalimetallsalzen der Fettsäuren, Alkalimetallalkylsulfaten und -sulfoestern, Polyoxyethylen-Polyoxypropylen-Copolymeren, Sorbitanfettsäureestern und polyoxyethylierten Analoga davon, Phospholipiden, Polyethylenglykolestern der Fettsäuren, polymerisierbaren Amphiphilen und oberflächenaktiven Systemen in Form von Multikompartimentvesikeln ausgewählte oberflächenaktive Mittel.

9. Kontrastmittel nach einem der Ansprüche 1 bis 5, wobei die Ölphase der Emulsion Sojaöl und Eigelbphospholipid umfasst und die wässrige Phase Glycerin und Wasser zur Injektion umfasst.

10. Kontrastmittel nach einem der vorhergehenden Ansprüche, wobei Mikroteilchen eines oder mehrerer bioverträglicher Mineralien in der Ölphase suspendiert sind.

11. Verfahren zur Herstellung eines Ultraschallkontrastmittels gemäß Definition im Anspruch 1, bei dem man entweder (i) eine Ölphase, die eine Lösung eines öllöslichen und im Wesentlichen wasserunlöslichen Gases, einer solchen flüchtigen Flüssigkeit oder eines solchen Gasvorläufers in wenigstens einer lipophilen Lösungsmittelkomponente umfasst, soweit erforderlich, in Gegenwart eines biokompatiblen Emulgiermittels, in einer wässrigen Phase emulgiert, wobei eine Öl-in-Wasser-Emulsion erhalten wird, oder (ii) ein gewünschtes öllösliches und im Wesentlichen wasserunlösliches Gas, eine solche flüchtige Flüssigkeit oder einen solchen Gasvorläufer in die Ölphase einer vorgebildeten Öl-in-Wasser-Emulsion einverleibt.

12. Verfahren zur Erzeugung verstärkter Abbildungen eines menschlichen oder tierischen Körpers, dem man zuvor ein Konrastmittel nach einem der Ansprüche 1 bis 10 verabreicht hat, wobei man ein Ultraschallbild wenigstens eines Teils des Körpers erzeugt.

## Revendications

1. Agent de contraste ultrasonore constitué d'une émulsion huile-dans-eau biocompatible et d'un ou plusieurs agent(s) d'émulsification biocompatible(s) quelconque(s) nécessaire(s), la phase huileuse de ladite émulsion étant essentiellement constituée d'une solution dans un solvant lipophile d'une substance soluble dans l'huile et sensiblement insoluble dans l'eau, choisie parmi des gaz, des fluides volatils et des précurseurs de gaz.

2. Agent de contraste selon la revendication 1 dans lequel le gaz ou le fluide volatil est choisi parmi les gaz inertes, les hydrocarbures éventuellement halogénés ou autrement substitués, les éthers aliphatiques ou cycliques, les silanes, les arsines et les halogénures de soufre.

3. Agent de contraste selon la revendication 2 dans lequel le gaz ou le fluide volatil est un perfluoroalcane.

4. Agent de contraste selon la revendication 2 dans lequel le gaz ou le fluide volatil est choisi parmi le xénon, le n-pentane, le furane, le tétraméthylsilane, l'hexafluorure de soufre et le perfluoro-n-butane.

5. Agent de contraste selon la revendication 1 dans lequel le précurseur de gaz est un carbonate organique ; une pyrazoline, un pyrazole, une triazoline, une diazocétone, un sel de diazonium, un tétrazole, un azide ou un mélange azide/carbonate ; une cétone cyclique, une lactone ou un carbonate, photolysable ; un peracide ; un acide carboxylique thermodégradable ; un méthylène-diester ou du peroxyde d'hydrogène, dégradable par voie enzymatique.

6. Agent de contraste selon la revendication 1 dans lequel le solvant lipophile est choisi parmi les perfluorocarbones aliphatiques et cycloaliphatiques et leurs analogues bromés, les perfluoroamines aliphatiques et cycloaliphatiques, les éthers perfluorés et les mélanges de n'importe lesquels des précédents.

7. Agent de contraste selon la revendication 6 dans lequel le solvant lipophile est le bromure de perfluorooctyle, la perfluorodécaline ou un mélange de perfluorodécaline et de perfluorotripropylamine.

8. Agent de contraste selon l'une quelconque des revendications 1 à 7 contenant en tant qu'agent d'émulsification un ou plusieurs tensioactifs choisis parmi les sels de métaux alcalins d'acides gras, les alkylsulfates et esters sulfonés de métaux alcalins, les copolymères de polyoxyéthylène-polyoxypropylène, les esters d'acide gras de sorbitane et leurs analogues polyéthoxylés, les phospholipides, les esters de polyéthylèneglycol d'acides gras, les amphiphiles polymérisables et les systèmes de tensioactif de type vésicule multicompartimentée.

9. Agent de contraste selon l'une quelconque des revendications 1 à 5 dans lequel la phase huileuse de l'émulsion comprend de l'huile de soja et du phospholipide de jaune d'oeuf, et la phase aqueuse comprend de la glycérine et de l'eau pour injection.

10. Agent de contraste selon l'une quelconque des revendications précédentes dans lequel des microparticules d'un ou plusieurs minéraux biotolérables sont mis en suspension dans la phase huileuse.

11. Procédé de préparation d'un agent de contraste ultrasonore tel que défini dans la revendication 1 qui comprend soit (i) l'émulsification d'une phase huileuse comprenant une solution d'un gaz, fluide volatil ou précurseur de gaz, soluble dans l'huile et sensiblement insoluble dans l'eau, dans au moins un composant solvant lipophile dans une phase aqueuse, si nécessaire en présence d'un agent d'émulsification biocompatible, de façon à former une émulsion huile-dans-eau, soit (ii) l'incorporation d'un gaz, fluide volatil ou précurseur de gaz, soluble dans l'huile et sensiblement insoluble dans l'eau, désiré, dans la phase huileuse d'une émulsion huile-dans-eau préformée.

12. Procédé pour générer des images améliorées d'un corps animal humain ou non humain auquel a été préalablement administré un agent de contraste selon l'une quelconque des revendications 1 à 10, lequel procédé comprend le fait de générer une image ultrasonique d'au moins une partie dudit corps.
